# EUROPÄISCHE PATENTSCHRIFT

(11) **EP 3 293 521 B1**
(45) Veröffentlichungstag und Bekanntmachung des Hinweises auf die Patenterteilung: **02.01.2019**
(21) Anmeldenummer: 16187839.2
(22) Anmeldetag: 08.09.2016
(51) Int. Cl.: G01N 33/92

(54) **HERSTELLUNG LIPÄMISCHER PLASMA- ODER SERUMPROBEN FÜR DIE BESTIMMUNG EINER LIPIDINTERFERENZ**
PRODUCTION OF LIPAEMIC PLASMA OR SERUM SAMPLES FOR DETECTING LIPID INTERFERENCE
FABRICATION D'ECHANTILLONS DE SERUM OU DE PLASMA LIPEMIQUE POUR LA DETERMINATION D'UNE INTERFERENCE LIPIDIQUE

(43) Veröffentlichungstag der Anmeldung: 14.03.2018
(73) Patentinhaber: Siemens Healthcare Diagnostics Products GmbH, 35041 Marburg (DE)
(72) Erfinder: Patzke, Juergen, 35043 Marburg (DE)

(56) Entgegenhaltungen:
- Heller Christian et al.: "Lipid Interference in the Determination of the Concentration of Haemoglobin in Plasma Using the AC A SX Analyzer 1 )", Eur J Clin Chem Clin Biochem, Oktober 1996 (1996-10), Seiten 811-816, XP055344645, Gefunden im Internet: URL:http://edoc.hu-berlin.de/oa/degruyter/ cclm.1996.34.10.811.pdf [gefunden am 2017-02-10]
- JOSHUA A BORNHORST ET AL: "Assay-Specific Differences in Lipemic Interference in Native and Intralipid-Supplemented Samples", CLINICAL CHEMISTRY., Bd. 50, Nr. 11, 16. September 2004 (2004-09-16), Seiten 2197-2201, XP055344471, WASHINGTON, DC. ISSN: 0009-9147, DOI: 10.1373/clinchem.2004.040683

## Beschreibung

Die vorliegende Erfindung liegt auf dem Gebiet der in vitro-Diagnostik und betrifft ein Verfahren zur Herstellung lipämischer Plasma- oder Serumproben und deren Verwendung zur Feststellung einer Lipidinterferenz bei der quantitativen Bestimmung der Menge oder der Aktivität eines Analyten in einer Plasma- oder Serumprobe.

Die Bestimmung klinisch relevanter Parameter in Plasma- und Serumproben kann durch erhöhte Triglyzeridkonzentrationen (Lipämie) signifikant beeinflusst werden. Lipämische Proben mit erhöhter Triglyzeridkonzentration treten verhältnismäßig häufig auf und können beispielsweise durch fettreiche Ernährung, Diabetes mellitus, chronisches Nierenversagen, Bauchspeicheldrüsenentzündung, Lupus erythematosus, multiples Myelom oder die Einnahme von Medikamenten oder oralen Antikontrazeptiva verursacht werden.

Der interferierende Effekt erhöhter Triglyzeridkonzentrationen beruht in erster Linie auf der zum Teil mit bloßem Auge erkennbaren Trübung (Turbidität) der Proben, die eine erhöhte Lichtstreuung und Absorption zur Folge hat. Dieses Phänomen interferiert am meisten mit photometrischen Testsystemen. Ein weiterer interferierender Effekt ist, dass die Löslichkeit eines nachzuweisenden Analyten beeinträchtigt sein kann.

Moderne automatische Analysegeräte umfassen deshalb zunehmend sogenannte präanalytische Analyseeinheiten, in denen das Probenmaterial hinsichtlich etwaiger Interferenzsubstanzen, wie Lipiden, Hämoglobin und Bilirubin, analysiert wird, bevor die eigentliche Bestimmung eines oder mehrerer spezifischer Analyten durchgeführt wird. Werden kritische Mengen einer oder mehrerer Interferenzsubstanzen in einer Probe festgestellt, können beispielsweise die für die Probe erhaltenen Testergebnisse mit einem Warnhinweis versehen werden, so dass ein Benutzer informiert ist, dass hier möglicherweise ein falsches Ergebnis gemessen wurde.
Da die Triglyzeridkonzentration, die eine signifikante Interferenz verursacht, für jeden Test unterschiedlich ist und von dem verwendeten Analysegerät, den verwendeten Reagenzien etc. abhängt, ist es erforderlich, für jeden Test Interferenzstudien durchzuführen, um zu ermitteln, ab welcher Triglyzeridkonzentration ein spezifischer Test keine zuverlässigen Testergebnisse mehr liefert.

Problematisch ist, dass es bislang kein standardisiertes lipämisches Probenmaterial gibt, das für die Durchführung der Interferenzstudien benötigt wird. Bislang wird häufig humanen Plasmaproben oder Plasmapools Intralipid, eine Sojabohnen-Lipid-Emulsion zugesetzt, um lipämische Interferenzen zu simulieren. Allerdings wurde gefunden, dass der Zusatz von Intralipid keine allgemein anwendbare Methode zur Lipidinterferenzbestimmung ist, weil die durch Intralipid hervorgerufenen Interferenzen nicht in allen Fällen mit den in nativen lipämischen Proben vorkommenden Interferenzen korrelieren (Bornhorst, J.A. et al., Assay-specific differences in lipemic interference in native and Intralipidsupplemented samples. Clin. Chem. 2004, 50(11): 2197-2201). Daher ist es bevorzugt, lipämische Spenderproben für Interferenzstudien einzusetzen. Heller et al., Lipid Interference in the Determination of the Concentration of Haemoglobin in Plasma Using the AC A SX Analyzer 1, Eur J Clin Chem Clin Biochem, Oktober 1996 (1996-10), Seiten 811-816; offenbart ein Verfahren zur Herstellung einer lipämischen Plasma- oder Serumprobe.

Allerdings sind ausreichende Mengen nativer lipämischer Patientenproben, insbesondere solche mit außergewöhnlich hohen Triglyceridkonzentrationen (> 500 mg/dL) nur mit sehr großem Aufwand erhältlich, da extrem große Spenderkollektive untersucht werden müssten.

Der vorliegenden Erfindung liegt damit die Aufgabe zugrunde, ein einfacheres Verfahren zur Bereitstellung lipämischer Plasma- oder Serumproben aufzufinden.
Die Aufgabe wird erfindungsgemäß dadurch gelöst, dass eine lipidhaltige Plasma- oder Serumprobe zur Gewinnung einer Lipid-angereicherten Phase zentrifugiert wird und die Lipidangereicherte Phase dann einer Plasma- oder Serumprobe zugesetzt wird.

Dies hat den Vorteil, dass jede gewünschte Triglyceridkonzentration erzeugt werden kann, ohne dass artifizielle Substanzen wie Intralipid verwendet werden müssen.

Gegenstand der vorliegenden Erfindung ist also ein Verfahren zur Herstellung einer lipämischen Plasma- oder Serumprobe. Das Verfahren umfasst die folgenden Schritte:
(a) Zentrifugation einer ersten Teilmenge einer lipidhaltigen Plasma- oder Serumprobe zur Trennung eines lipidhaltigen Überstandes von einer Lipid-abgereicherten Phase;
(b) Abtrennung des lipidhaltigen Überstandes und
(c) Vermischen des lipidhaltigen Überstandes mit einer zweiten Teilmenge derselben lipidhaltigen Plasma- oder Serumprobe.
Die Begriffe "Triglycerid(e)" und "Lipid(e)" werden synonym verwendet.
Unter dem Begriff "lipämische Plasma- oder Serumprobe" ist eine Plasma- oder Serumprobe eines einzelnen Spenders oder eine Mischung (Pool) von Plasma- oder Serumproben mehrerer Spender zu verstehen, deren Triglyceridkonzentration oberhalb des Referenzbereiches liegt, d.h. die 150 mg/dL (1,71 mmol/L) oder mehr beträgt.
Unter dem Begriff "lipidhaltige Plasma- oder Serumprobe" ist eine Plasma- oder Serumprobe eines einzelnen Spenders oder eine Mischung (Pool) von Plasma- oder Serumproben mehrerer Spender zu verstehen, deren Triglyceridkonzentration innerhalb oder oberhalb des Referenzbereiches von ≤150 mg/dL (1,71 mmol/L) liegt.

Die Erfindung bezieht sich insbesondere auf humane Plasma- und Serumproben. Ein analoges Vorgehen ist jedoch grundsätzlich auch für tierische Plasma- und Serumproben anwendbar.

In der erfindungsgemässen Ausführungsform wird eine lipidhaltige Plasma- oder Serumprobe geteilt, und es wird in Schritt (a) des Verfahrens eine erste Teilmenge der lipidhaltigen Plasma- oder Serumprobe zentrifugiert und dann in Schritt (b) der lipidhaltige Überstand abgetrennt, und dann wird in Schritt (c) der lipidhaltige Überstand mit einer zweiten Teilmenge derselben lipidhaltigen Plasma- oder Serumprobe vermischt. Dies hat den Vorteil, dass in dieser spezifischen Probe, die beispielsweise eine definierte Analytkonzentration oderaktivität aufweist, lediglich der Lipidanteil erhöht wird, während die übrige Probenzusammensetzung unverändert bleibt. Weiterhin ist vorteilhaft, dass die Interferenz, die durch das Lipid dieser spezifischen Probe hervorgerufen wird, untersucht werden kann. Somit könnte in einer Studie mit mehreren verschiedenen lipämischen Proben auch die Variabilität dieser Proben bezüglich der Interferenz in die finale Festlegung des Interferenzgrenzwertes mit einfließen.
Alternativ kann der abgetrennte lipidhaltige Überstand in Schritt (c) mit einer Teilmenge der Lipid-abgereicherten Phase der zentrifugierten Probe vermischt werden.
Das Mischungsverhältnis von lipidhaltigem Überstand und Plasma oder -Serumprobe hängt selbstverständlich von der Triglyceridkonzentration der Ausgangsmaterialien sowie der gewünschten Triglyceridkonzentration ab.

Die Zentrifugation der lipidhaltigen Plasma- oder Serumprobe in Schritt (a) erfolgt vorzugsweise für mindestens 10 Minuten bei mindestens 2.000×g. Die Anwendung längerer Zentrifugationszeiten und höherer Zentrifugalkräfte ist durchaus möglich, wie beispielsweise für 10 Minuten bei 15.000×g oder für 60 Minuten bei 82.000×g oder auch für 60 Minuten bei 133.000×g.

Die Zentrifugation einer lipidhaltigen Plasma- oder Serumprobe führt zur mit bloßem Auge sichtbaren Separation eines trüben, lipidhaltigen Überstandes von einer darunterliegenden klaren, Lipid-abgereicherten Phase. Die Abtrennung des lipidhaltigen Überstandes kann beispielsweise erfolgen, indem mit einer Pipettiernadel vorsichtig durch den lipidhaltigen Überstand gestochen wird und die Lipid-abgereicherte Phase vorsichtig abgesaugt wird, so dass nur der lipidhaltige Überstand im Gefäß verbleibt. Durch Zugabe einer Plasma- oder Serumprobe in das Gefäß und Schütteln wird die Probe mit dem lipidhaltigen Überstand vermischt. Andere bekannte Verfahren zur Homogenisierung, wie z.B. eine Ultraschallbehandlung können auch angewendet werden.

Ein weiterer Gegenstand der vorliegenden Erfindung ist die Verwendung einer mit einem erfindungsgemäßen Verfahren hergestellten lipämischen Plasma- oder Serumprobe in einem Verfahren zur Feststellung einer Lipidinterferenz in einem Verfahren zur quantitativen Bestimmung der Menge oder der Aktivität eines Analyten in einer Plasma- oder Serumprobe.

Bevorzugterweise wird eine erfindungsgemäß hergestellte lipämische Plasma- oder Serumprobe in einem Verfahren zur Feststellung einer Lipidinterferenz in einem Verfahren zur quantitativen Bestimmung der Menge oder der Aktivität eines Analyten in einer Plasma- oder Serumprobe verwendet, wobei das Verfahren zur Feststellung einer Lipidinterferenz folgende Schritte umfasst:
(a) Bereitstellen eines ersten Testansatzes durch Vermischen mindestens eines analytspezifischen Nachweisreagenzes mit einer nicht-lipämischen Plasma- oder Serumprobe mit einer Analytkonzentration oder - aktivität und Messen eines ersten Testergebnisses;
(b) Bereitstellen eines zweiten Testansatzes durch Vermischen desselben mindestens einen analytspezifischen Nachweisreagenzes mit einer lipämischen Plasma- oder Serumprobe derselben Analytkonzentration oder -aktivität und Messen eines zweiten Testergebnisses;
(c) Feststellen einer Differenz zwischen dem ersten und zweiten Testergebnis; und
(d) Feststellen einer Lipidinterferenz, wenn die Differenz zwischen dem ersten und zweiten Testergebnis eine vorbestimmte Toleranzgrenze überschreitet, beispielsweise wenn die Differenz zwischen dem ersten und zweiten Testergebnis 5 % oder mehr beträgt.

Auf diese Weise kann für jeden analytischen Test bestimmt werden, ab welcher Triglyzeridkonzentration der Test keine zuverlässigen Testergebnisse mehr liefert.

Unter einem "Analyten" ist eine in einem Probenmaterial (hier Plasma oder Serum) zu detektierende Substanz oder eine messbare Eigenschaft des Probenmaterials, die durch eine Mehrzahl von Substanzen beeinflusst wird, zu verstehen. Ein Analyt kann beispielsweise ein Peptid, ein Protein, ein Polysaccharid oder eine Nukleinsäure sein, insbesondere ein Protein oder Proteinkomplex mit einer bestimmten biologischen Funktion, wie beispielsweise Immunglobuline, Zytokine, Rezeptoren, Enzyme, Hormone, Krebsantigene, gewebespezifische Antigene, Blutgerinnungfaktoren, Antigene mikrobieller Krankheitserreger etc. Eine typische durch eine Mehrzahl von Substanzen beeinflusste Eigenschaft einer Plasmaprobe ist beispielsweise die Gerinnungszeit. Gerinnungsteste ermöglichen die Messung der Aktivität eines einzelnen oder mehrerer Gerinnungsfaktoren durch die Messung der Fibrinbildungsgeschwindigkeit in vitro.

Bei dem Verfahren zur quantitativen Bestimmung der Menge oder der Aktivität eines Analyten in einer Plasma- oder Serumprobe kann es sich um jedes denkbare Testprinzip handeln, wie beispielsweise einen partikelverstärkten Immuntest, einen chromogenen Test oder einen Gerinnungstest.

Ein analytspezifisches Nachweisreagenz enthält ein oder mehrere Substanzen, die den Nachweis eines spezifischen Analyten oder einer anderen messbaren Eigenschaft der Probe ermöglichen, beispielsweise einen oder mehrere Antikörper oder Antigene, chromogene Peptidsubstrate und/oder Enzymaktivatoren. Es kann beispielsweise vorgesehen sein, ein flüssiges Nachweisreagenz, das die analytspezifischen Substanzen in gelöster Form enthält, mit der Probe zu vermischen. Alternativ kann vorgesehen sein, dass ein Nachweisreagenz aus einer Festphase besteht, die mit einer oder mehreren analytspezifischen Substanzen beschichtet ist.

Die Messung der Testergebnisse kann je nach angewandtem Testprinzip erfolgen, beispielsweise spektrophotometrisch, turbidimetrisch, nephelometrisch, luminometrisch, fluorometrisch, radiometrisch etc.

Zur Feststellung einer Lipidinterferenz, also einer signifikanten Beeinträchtigung der Messgenauigkeit eines Testverfahrens, wird dasselbe Testverfahren sowohl mit einer nicht-lipämischen Plasma- oder Serumprobe (mit einer Triglyzeridkonzentration von ≤ 150 mg/dL) als Referenzprobe als auch mit mindestens einer erfindungsgemäß hergestellten lipämischen Plasma- oder Serumprobe (mit einer Triglyzeridkonzentration von > 150 mg/dL) durchgeführt. Sofern die beiden Proben dieselbe Analytkonzentration oderaktivität aufweisen, ist eine Differenz zwischen dem ersten und zweiten Testergebnis auf die erhöhte Triglyzeridkonzentration der lipämischen Probe zurückzuführen. Vorzugsweise handelt es sich bei der Referenzprobe um die Lipid-abgereicherte Phase der lipidhaltigen Probe, die für die Herstellung der lipämischen Probe zentrifugiert wurde.

Eine Lipidinterferenz liegt dann vor, wenn die Differenz zwischen dem ersten und zweiten Testergebnis eine vorbestimmte Toleranzgrenze überschreitet, beispielsweise wenn die relative Differenz zwischen dem ersten und zweiten Testergebnis 5 % oder mehr beträgt. Es kann auch eine maximale absolute Analytdifferenz als Toleranzgrenze definiert werden.

Vorzugsweise werden für eine Lipidinterferenzstudie für jeden Probentyp mehrere Proben gemessen, vorzugweise 10 bis 20, so dass eine statistische Auswertung durchgeführt werden kann. Vorzugsweise werden 10 bis 20 nicht-lipämische Proben mit normaler Analytkonzentration oder -aktivität und dieselbe Anzahl lipämischer Proben mit normaler Analytkonzentration oder -aktivität gemessen. Zusätzlich oder alternativ kann eine Anzahl nicht-lipämischer Proben und parallel dieselbe Anzahl lipämischer Proben mit Analytkonzentrationen oder - aktivitäten gemessen werden, welche den Messbereich des Testverfahrens abdecken. Weiterhin können auch unterschiedliche lipämische Probentypen mit normaler, verminderter oder erhöhter Analytkonzentration oder - aktivität gemessen werden, die unterschiedliche Triglyzeridkonzentrationen oberhalb des Referenzbereiches aufweisen, also Konzentrationen zwischen 150 und 3000 mg/dL.

Vorzugsweise werden für die Feststellung einer Lipidinterferenz eine lipämische Plasma- oder Serumprobe und eine nicht-lipämische Plasma- oder Serumprobe verwendet, die aus derselben lipidhaltigen Plasma- oder Serumprobe als Ausgangsmaterial hergestellt sind. Dies hat den Vorteil, dass in den beiden Proben, bis auf den Lipidanteil, dessen Einfluss untersucht werden soll, die Probenzusammensetzung und damit auch die Analytkonzentration identisch ist.

Weiterhin bevorzugterweise kann die nicht-lipämische Plasma- oder Serumprobe mit einem Verfahren umfassend die folgenden Schritte hergestellt sein:
(a) Zentrifugation einer lipidhaltigen Plasma- oder Serumprobe und Isolierung der Lipid-abgereicherten Phase vom lipidhaltigen Überstand.

Dies hat den Vorteil, dass die bei der Herstellung des lipidhaltigen Überstandes, der für die Herstellung einer lipämischen Probe vorgesehen ist, als Nebenprodukt anfallende Lipid-abgereicherte Phase verwendet werden kann, so dass ein vorhandenes Ausgangsmaterial weitestgehend verschwendungsfrei eingesetzt werden kann.

Eine erfindungsgemäß hergestellte lipämische Plasma- oder Serumprobe kann auch in einem Verfahren zur Feststellung einer Lipidinterferenz in einem Verfahren zur quantitativen Bestimmung der Menge oder der Aktivität eines Analyten in einer Plasma- oder Serumprobe verwendet werden, wobei das Verfahren zur Feststellung einer Lipidinterferenz folgende Schritte umfasst:
(a) Bereitstellen eines ersten Testansatzes durch Vermischen mindestens eines analytspezifischen Nachweisreagenzes mit einer ersten Teilmenge einer nicht-lipämischen Plasma- oder Serumprobe und Messen eines ersten Testergebnisses;
(b) Bereitstellen eines zweiten Testansatzes durch Vermischen desselben mindestens einen analytspezifischen Nachweisreagenzes mit der Lipid-abgereicherten Phase einer zweiten Teilmenge derselben nicht-lipämischen Plasma- oder Serumprobe, die zuvor für mindestens 10 Minuten bei mindestens 2000g zentrifugiert worden war, und Messen eines zweiten Testergebnisses; und
(c) Feststellen einer ersten Differenz zwischen dem ersten und zweiten Testergebnis; und
(d) Bereitstellen eines dritten Testansatzes durch Vermischen desselben mindestens einen analytspezifischen Nachweisreagenzes mit einer ersten Teilmenge einer lipämischen Plasma- oder Serumprobe und Messen eines dritten Testergebnisses;
(e) Bereitstellen eines vierten Testansatzes durch Vermischen desselben mindestens einen analytspezifischen Nachweisreagenzes mit der Lipid-abgereicherten Phase einer zweiten Teilmenge derselben lipämischen Plasma- oder Serumprobe, die zuvor für mindestens 10 Minuten bei mindestens 2000g zentrifugiert worden war, und Messen eines vierten Testergebnisses; und
(f) Feststellen einer zweiten Differenz zwischen dem dritten und vierten Testergebnis; und
(g) Feststellen einer Lipidinterferenz, wenn die Abweichung zwischen der ersten und zweiten Differenz eine vorbestimmte Toleranzgrenze überschreitet, beispielsweise wenn die Abweichung zwischen der ersten und zweiten Differenz, die z.B. jeweils als relative Differenzen ausgedrückt sind, 5 % oder mehr überschreitet.

Eine lipämische Plasma- oder Serumprobe kann auch in einer Ausführungsform des Verfahrens zur Feststellung einer Lipidinterferenz in einem Verfahren zur quantitativen Bestimmung der Menge oder der Aktivität eines Analyten in einer Plasma- oder Serumprobe verwendet werden, wobei die Ausführungsform des Verfahrens zur Feststellung einer Lipidinterferenz folgende Schritte umfasst:
(a) Bereitstellen mehrerer erster Testansätze jeweils durch Vermischen mindestens eines analytspezifischen Nachweisreagenzes mit jeweils einer ersten Teilmenge nicht-lipämischer Plasma- oder Serumproben und Messen mehrerer erster Testergebnisse;
(b) Bereitstellen mehrerer zweiter Testansätze jeweils durch Vermischen desselben mindestens einen analytspezifischen Nachweisreagenzes mit jeweils der Lipid-abgereicherten Phase einer zweiten Teilmenge derselben nicht-lipämischen Plasma- oder Serumproben, die zuvor für mindestens 10 Minuten bei mindestens 2000g zentrifugiert worden waren, und Messen mehrerer zweiter Testergebnisse; und
(c) Feststellen einer ersten Differenz zwischen dem jeweiligen ersten und zweiten Testergebnis und Bilden eines Mittelwerts aller festgestellten ersten Differenzen; und
(d) Bereitstellen eines dritten Testansatzes durch Vermischen desselben mindestens einen analytspezifischen Nachweisreagenzes mit einer ersten Teilmenge einer lipämischen Plasma- oder Serumprobe und Messen eines dritten Testergebnisses;
(e) Bereitstellen eines vierten Testansatzes durch Vermischen desselben mindestens einen analytspezifischen Nachweisreagenzes mit der Lipid-abgereicherten Phase einer zweiten Teilmenge derselben lipämischen Plasma- oder Serumprobe, die zuvor für mindestens 10 Minuten bei mindestens 2000g zentrifugiert worden war, und Messen eines vierten Testergebnisses; und
(f) Feststellen einer zweiten Differenz zwischen dem dritten und vierten Testergebnis; und
(g) Bilden einer korrigierten zweiten Differenz durch Abziehen des Mittelwerts aller festgestellten ersten Differenzen; und
(h) Feststellen einer Lipidinterferenz, wenn die korrigierte zweite Differenz eine vorbestimmte Toleranzgrenze überschreitet, beispielsweise wenn die korrigierte Differenz einen zuvor bestimmten Vertrauensbereich von 10 % überschreitet.

Dies hat den Vorteil, dass etwaige Effekte des Zentrifugationsverfahrens, das zur Herstellung der Lipid-abgereicherten Phase einer Probe angewendet wird, die eine Veränderung der Analytkonzentration bewirken können, erkannt werden können. Wenn das Zentrifugationsverfahren selbst den Analytgehalt der Probe verändert, muss dieser Effekt bei der Ermittlung einer Interferenz berücksichtigt werden. Es ist zum Beispiel bei einem Gerinnungstest vorstellbar, dass durch die Zentrifugation wichtige Vesikel-gebundene Faktoren sedimentiert werden und sich allein dadurch die Gerinnungszeit verändert. Aus diesem Grund wird das Zentrifugationsverfahren nicht nur bei lipämischen Proben, sondern auch bei nicht-lipämischen Proben angewendet. Wenn zum Beispiel sowohl bei lipämischen Proben wie auch bei nicht-lipämischen Proben jeweils eine Abweichung des Analytgehaltes von relativ 10% in der Lipid-abgereicherten Phase gegenüber der nicht zentrifugierten Probe festgestellt wird, ist diese auf die Zentrifugation zurückzuführen. Eine Triglyzerid-Interferenz liegt dann nicht vor. Wenn das Zentrifugationsverfahren eine Abweichung von + 10 % im Analytgehalt von nicht-lipämischen Proben erzeugt und in lipämischen Proben eine Abweichung von + 20% beobachtet wird, kann man daraus auf eine 10 %ige Interferenz durch die Triglyzeride schliessen.

Da sowohl das Ausmaß der Interferenz als auch der mögliche Einfluss des Zentrifugationsverfahrens bei einzelnen nicht-lipämischen und lipämischen Proben unterschiedlich stark sein kann, ist es sinnvoll, lipämische Proben mit unterschiedlichen Triglyzeridgehalten und unterschiedlichen Analytgehalten und nicht-lipämische Proben mit unterschiedlichen Analytgehalten zu messen.

### Figurenbeschreibung

- FIG. 1: Bestimmung der Lipidinterferenz in einem Verfahren zur Bestimmung der APTT;
- FIG. 2: Bestimmung der Lipidinterferenz in einem Verfahren zur Bestimmung von Protein C.

Die folgenden Ausführungsbeispiele dienen der Veranschaulichung des erfindungsgemäßen Verfahrens und sind nicht als Einschränkung zu verstehen.

### BEISPIELE

### BEISPIEL 1: Bestimmung der Lipidinterferenz in einem Verfahren zur Bestimmung der APTT

### 1a) Erfindungsgemäße Herstellung lipämischer Plasmaproben für die Bestimmung der Lipidinterferenz in einem Verfahren zur Bestimmung der APTT mit Dade Actin Reagenz

Es wurden 7 native lipämische Citrat-Plasma-Proben (Triglyzeridgehalt > 150 mg/dL) von 7 Spendern verwendet. Die Bestimmung des Triglyzeridgehaltes erfolgte unter Verwendung des TRIG-Tests und mit dem Dimension Vista System (Siemens Healthcare, Newark, USA). Etwa 18 bis 20 mL dieser Proben wurden für 1 Stunde in einer Ultrazentrifuge (Thermo Scientific Sorval WX Ultra, Rotor T-1250, ThermoFisher, Hanau, Germany) bei 33.200 UPM zentrifugiert, was einer g-Zahl von 133.000 entspricht. Der oberste, lipidreiche Überstand wurde mit Hilfe einer Pipette vorsichtig, unter Vermeidung einer Vermischung mit der darunterliegenden, Lipid-abgereicherten Phase entnommen. Je nach Probe wurden zwischen 0,7 und 3,0 mL dieses lipidreichen Überstandes mit einer nicht zentrifugierten Teilmenge der nativen Citrat-Plasma-Probe desselben Spenders und teilweise mit einem nicht-lipämischen Faktor-Mangelplasma (Triglyzeridgehalt typischerweise ca. 100 mg/dL) gemischt. Das Gesamtvolumen der auf diese Weise hergestellten lipämischen Proben betrug etwa 12 mL. Der Triglyzeridgehalt der so hergestellten Proben wurde nochmals gemessen und lag zwischen 234 mg/dL und 1007 mg/dL.

Tabelle 1 enthält die finale Triglyzeridkonzentration der erfindungsgemäß hergestellten lipämischen Proben-Nr. L3-L9 und drei weiterer nativer lipämischer Proben (Proben-Nr. L1, L2 und L10) sowie das Mischungsverhältnis der Menge von nativer (nicht zentrifugierter, unbehandelter Plasmaprobe) des Spenders, lipidhaltigem Überstand, Mangelplasma und einer Heparinlösung. Die Vermischung mit Mangelplasma oder Heparinlösung erfolgte zur Erweiterung des APTT-Messbereichs.

Tabelle 2 enthält die Zusammensetzung und die finale Triglyzeridkonzentration von den nicht-lipämischen Proben-Nr. NL1-NL12, die in der Interferenzstudie ebenfalls eingesetzt wurden.

**Tabelle 1: Zusammensetzung der lipämischen Proben**

| **Proben-Nr.** | **Zusammensetzung** | | | | | | |
|---|---|---|---|---|---|---|---|
| | Native Probenmenge | Lipid-haltiger Überstand | Plasma mit einem Mangel an | | | Heparinlösung | Finale Triglyzeridkonzentration |
| | | | FV | FVII | PC | | |
| | mL | mL | mL | mL | mL | mL | mg/dL |
| L1 | 12,0 | 0,0 | 0,0 | 0,0 | 0,0 | 0,0 | 237,5 |
| L2 | 12,0 | 0,0 | 0,0 | 0,0 | 0,0 | 0,0 | 399,5 |
| **L3** | 11,1 | **0,9** | 0,0 | 0,0 | 0,0 | 0,0 | 731,0 |
| **L4** | 3,0 | **3,0** | 0,0 | 6,0 | 0,0 | 0,0 | 316,5 |
| **L5** | 3,0 | **2,5** | 0,0 | 0,0 | 8,0 | 0,0 | 481,5 |
| **L6** | 6,5 | **0,7** | 0,0 | 5,0 | 0,0 | 0,0 | 1.006,6 |
| **L7** | 3,5 | **1,2** | 7,5 | 0,0 | 0,0 | 0,0 | 233,5 |
| **L8** | 4,0 | **2,0** | 6,0 | 0,0 | 0,0 | 0,0 | 405,5 |
| **L9** | 4,5 | **1,6** | 6,0 | 0,0 | 0,0 | 0,0 | 432,5 |
| L10 | 12,0 | 0,0 | 0,0 | 0,0 | 0,0 | 0,5 | 215,5 |

**Tabelle 2: Zusammensetzung der nicht-lipämischen Proben**

| **Proben-Nr.** | **Zusammensetzung** | | | | | | |
|---|---|---|---|---|---|---|---|
| | Native Probenm enge | Lipid-haltiger Überstand | Plasma mit einem Mangel an | | | Heparinlösung | Finale Triglyzeridkonzentration |
| | | | FV | FVII | PC | | |
| | mL | mL | mL | mL | mL | mL | mg/dL |
| NL1 | 12,0 | --- | 0,0 | 0,0 | 0,0 | 0,0 | 148,0 |
| NL2 | 12,0 | --- | 0,0 | 0,0 | 0,0 | 0,0 | 120,5 |
| NL3 | 12,0 | --- | 0,0 | 0,0 | 0,0 | 0,0 | 73,5 |
| NL4 | 12,0 | --- | 0,0 | 0,0 | 0,0 | 0,0 | 52,6 |
| NL5 | 6,0 | --- | 6,0 | 0,0 | 0,0 | 0,0 | 143,5 |
| NL6 | 12,0 | --- | 0,0 | 0,0 | 0,0 | 0,0 | 83,0 |
| NL7 | 1,2 | --- | 0,0 | 10,8 | 0,0 | 0,0 | 100,0 |
| NL8 | 3,0 | --- | 0,0 | 9,0 | 0,0 | 0,0 | 99,5 |
| NL9 | 7,0 | --- | 5,0 | 0,0 | 0,0 | 0,0 | 62,5 |
| NL10 | 6,0 | --- | 6,0 | 0,0 | 0,0 | 0,0 | 105,5 |
| NL11 | 6,6 | --- | 5,4 | 0,0 | 0,0 | 0,0 | 107,0 |
| NL12 | 12,0 | --- | 0,0 | 0,0 | 0,0 | 0,2 | 92,2 |
| NL13 | 11,5 | --- | 0,0 | 0,0 | 0,0 | 0,6 | 53,8 |
| NL14 | 12,0 | --- | 0,0 | 0,0 | 0,0 | 0,7 | 73,8 |

### 1b) Bestimmung der Lipidinterferenz in einem Verfahren zur Bestimmung der APTT mit Dade Actin Reagenz

Bei der aktivierten partiellen Thromboplastinzeit (APTT) wird eine Plasmaprobe mit Reagenzien vermischt, welche Phospholipide und einen Oberflächenaktivator enthalten (Dade Actin Reagenz, Siemens Healthcare, Marburg, Deutschland). Nach einer Inkubationszeit wird durch die Zugabe von CaCl₂ die Gerinnung ausgelöst. In dem hier beschriebenen Beispiel wurde die APTT auf dem Sysmex CS-5100 Analysegerät (Siemens Healthcare, Marburg, Deutschland) automatisch abgearbeitet. Die Gerinnungsreaktion wird photometrisch als Zunahme der Extinktion gemessen. Die Zeit bis zu einer bestimmten Zunahme der Extinktion ist die Gerinnungszeit in Sekunden, welche das Ergebnis des APTT-Tests darstellt.

Von allen der unter 1a) hergestellten Proben wurde eine Teilmenge zur Messung der APTT verwendet (Testergebnis 1). Eine zweite Teilmenge jeder der unter 1a) hergestellten Proben wurde für 1 Stunde bei 133.000×g zentrifugiert und die Lipid-abgereicherte Phase wurde für die APTT-Messung als Probe eingesetzt (Testergebnis 2). Sofern die Zentrifugation keinen Einfluss hat, sollte die Lipid-abgereicherte Phase des zentrifugierten Aliquots dieselbe Analytmenge wie die nicht zentrifugierte Probe enthalten. Die beiden Testergebnisse (Gerinnungszeiten in Sekunden) jeder Probe wurden miteinander verglichen, und es wurde die relative Differenz in % gebildet (100 x Testergebnis 1 - Testergebnis 2 / Testergebnis 2).

Aus den relativen Differenzen der Testergebnisse für die nicht-lipämischen Proben wurde der Mittelwert berechnet (hier: 0,6). Da die nicht-lipämischen Proben keine Interferenz durch Lipämie aufweisen, wird diese mittlere Differenz auf einen unspezifischen Einflussfaktor des Verfahrens (z.B. die Zentrifugation) zurückgeführt. Zur Korrektur dieses Effektes wurde für jede Probe eine korrigierte Differenz ermittelt, indem dieser Mittelwert von der relativen Differenz abgezogen wurde. Die korrigierte Differenz ist ein Maß für die durch Lipide verursachte Interferenz.

Tabelle 3 zeigt für jede Probe die Testergebnisse 1 und 2, die ermittelte relative Differenz und die korrigierte Differenz. Bei den Differenzwerten handelt es sich um gerundete Werte.

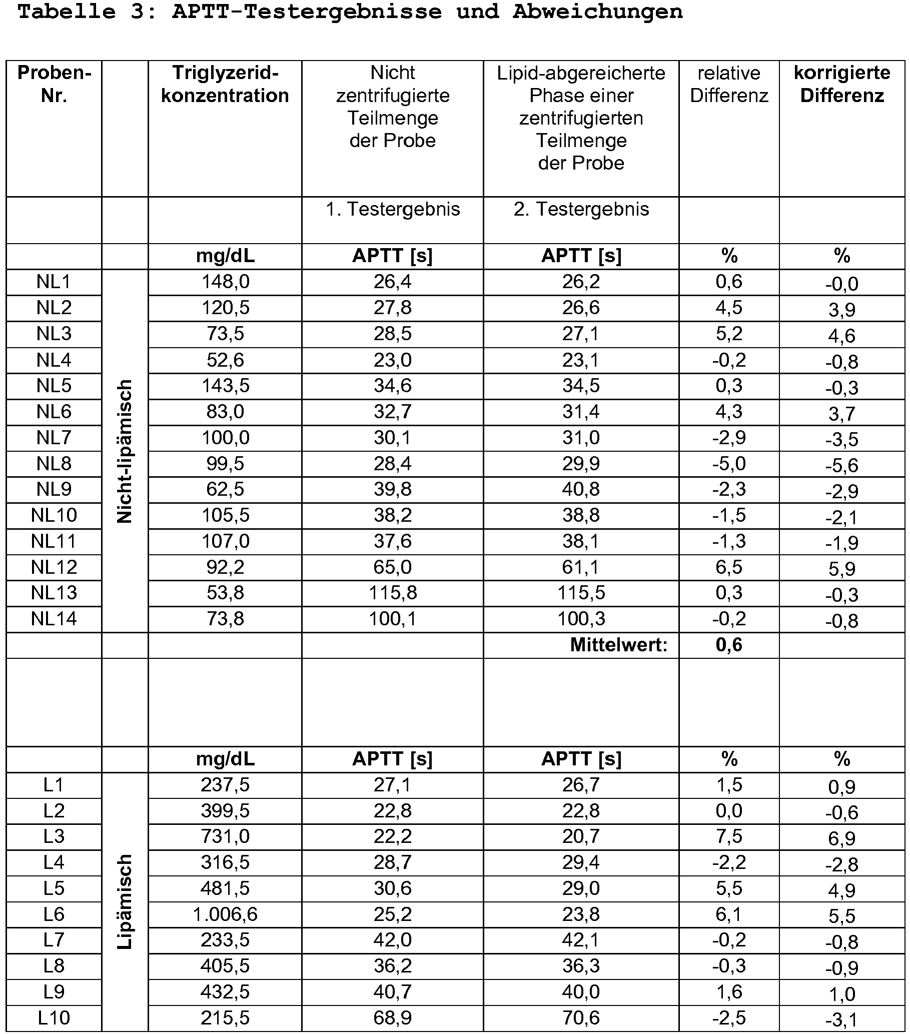

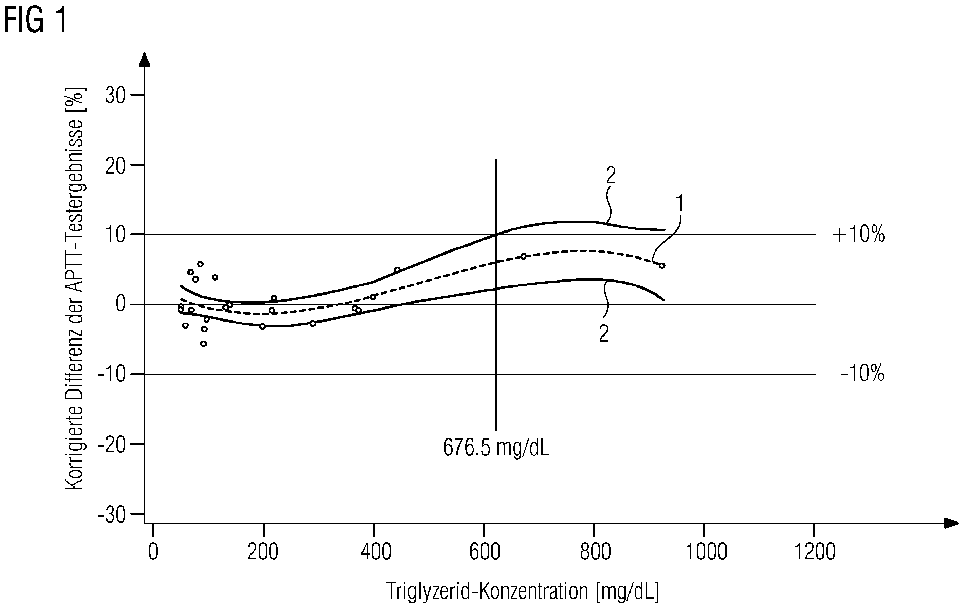

Die korrigierte Differenz, also die Abweichung der Testergebnisse der nicht zentrifugierten Proben von den Testergebnissen der zentrifugierten Proben(vermindert um den Mittelwert der Differenz der nicht-lipämischen Proben) wurde gegen die Triglyzeridkonzentration der nicht zentrifugierten Proben aufgetragen (Figur 1, Linie 1). Ein polynomisches Fitverfahren wurde angewandt und der dazugehörige Vertrauensbereich berechnet (Figur 1, Linien 2). Der Schnittpunkt der Grenze des Vertrauensbereiches mit dem Kriterium von 10 % relativer Abweichung kennzeichnet die Triglyzeridkonzentration, ab der eine Lipidinterferenz in dem getesteten APTT-Testverfahren zu erwarten ist (676,5 mg/dL).

### BEISPIEL 2: Bestimmung der Lipidinterferenz in einem Verfahren zur Bestimmung von Protein C

### 2a) Erfindungsgemäße Herstellung lipämischer Plasmaproben für die Bestimmung der Lipidinterferenz in einem Verfahren zur Bestimmung von Protein C mit dem Berichrom Protein Test

Es wurden 12 native lipämische Citrat-Plasma-Proben (Triglyzeridgehalt > 150 mg/dL) von 12 Spendern verwendet, und es wurde wie unter 1a) beschrieben verfahren. Der Triglyzeridgehalt der so hergestellten Proben wurde nochmals gemessen und lag zwischen 199 mg/dL und 1007 mg/dL.

Tabelle 4 enthält die finale Triglyzeridkonzentration der erfindungsgemäß hergestellten lipämischen Proben-Nr. L1-L12 sowie einer weiteren nativen lipämischen Probe (Proben-Nr. L13) sowie das Mischungsverhältnis der Menge von nativer (nicht zentrifugierter, unbehandelter Plasmaprobe) des Spenders, lipidhaltigem Überstand, Mangelplasma und einer Heparinlösung. Die Vermischung mit Mangelplasma erfolgte zur Erweiterung des Protein C-Messbereichs.

Tabelle 5 enthält die Zusammensetzung und die finale Triglyzeridkonzentration von den nicht-lipämischen Proben-Nr. NL1-NL12, die in der Interferenzstudie ebenfalls eingesetzt wurden.

**Tabelle 4: Zusammensetzung der lipämischen Proben**

| **Proben-Nr.** | **Zusammensetzung** | | | | | | |
|---|---|---|---|---|---|---|---|
| | Native Probenmenge | Lipid-haltiger Überstand | Plasma mit einem Mangel an | | | Heparinlösung | Finale Triglyzeridkonzentration |
| | | | FV | FVII | PC | | |
| | mL | mL | mL | mL | mL | mL | mg/dL |
| **L1** | 3,0 | **3,0** | 0,0 | 0,0 | 6,0 | 0,0 | 330,5 |
| **L2** | 3,7 | **2,1** | 0,0 | 0,0 | 5,7 | 0,0 | 351,0 |
| **L3** | 3,0 | **2,5** | 0,0 | 0,0 | 8,0 | 0,0 | 481,5 |
| **L4** | 2,8 | **1,7** | 0,0 | 0,0 | 7,5 | 0,0 | 566,5 |
| **L5** | 3,5 | **1,2** | 7,5 | 0,0 | 0,0 | 0,0 | 233,5 |
| **L6** | 5,5 | **2,3** | 0,0 | 0,0 | 3,8 | 0,0 | 545,5 |
| **L7** | 6,3 | **1,2** | 0,0 | 0,0 | 4,5 | 0,0 | 967,5 |
| **L8** | 4,3 | **0,9** | 6,8 | 0,0 | 0,0 | 0,0 | 199,0 |
| **L9** | 3,0 | **2,7** | 0,0 | 6,3 | 0,0 | 0,0 | 369,5 |
| **L10** | 4,5 | **1,6** | 6,0 | 0,0 | 0,0 | 0,0 | 432,5 |
| **L11** | 11,1 | **0,9** | 0,0 | 0,0 | 0,0 | 0,0 | 731,0 |
| **L12** | 6,5 | **0,7** | 0,0 | 5,0 | 0,0 | 0,0 | 1.006,6 |
| L13 | 12,0 | 0,0 | 0,0 | 0,0 | 0,0 | 0,0 | 237,5 |

**Tabelle 5: Zusammensetzung der nicht-lipämischen Proben**

| **Proben-Nr.** | **Zusammensetzung** | | | | | | |
|---|---|---|---|---|---|---|---|
| | Native Probenmenge | Lipid-haltiger Überstand | Plasma mit einem Mangel an | | | Heparinlösung | Finale Triglyzeridkonzentration |
| | | | FV | FVII | PC | | |
| | mL | mL | mL | mL | mL | mL | mg/dL |
| NL1 | 3,0 | --- | 0,0 | 0,0 | 9,0 | 0,0 | 91,0 |
| NL2 | 4,0 | --- | 0,0 | 0,0 | 8,0 | 0,0 | 95,5 |
| NL3 | 4,0 | --- | 0,0 | 0,0 | 8,0 | 0,0 | 103,0 |
| NL4 | 4,0 | --- | 0,0 | 0,0 | 8,0 | 0,0 | 87,5 |
| NL5 | 7,0 | --- | 5,0 | 0,0 | 0,0 | 0,0 | 62,5 |
| NL6 | 11,5 | --- | 0,0 | 0,0 | 0,0 | 0,6 | 53,8 |
| NL7 | 6,0 | --- | 0,0 | 0,0 | 6,0 | 0,0 | 88,0 |
| NL8 | 7,0 | --- | 0,0 | 0,0 | 5,0 | 0,0 | 92,0 |
| NL9 | 6,0 | --- | 6,0 | 0,0 | 0,0 | 0,0 | 105,5 |
| NL10 | 12,0 | --- | 0,0 | 0,0 | 0,0 | 0,0 | 83,0 |
| NL11 | 12,0 | --- | 0,0 | 0,0 | 0,0 | 0,0 | 120,5 |
| NL12 | 12,0 | --- | 0,0 | 0,0 | 0,0 | 0,0 | 73,5 |

### 2b) Bestimmung der Lipidinterferenz in einem Verfahren zur Bestimmung von Protein C mit dem Berichrom Protein C Test

Im Testansatz wird eine Plasmaprobe mit einem Schlangengiftaktivator inkubiert, wodurch Protein C aktiviert wird. Weiterhin wird ein chromogenes Peptidsubstrat zugegeben, welches durch aktiviertes Protein C gespalten wird. Durch diese Reaktion wird eine Extinktionszunahme erreicht, welche bei 405 nm gemessen wird. Die Extinktionszunahme wird anhand einer Kalibrationskurve in das Ergebnis Protein C (% der Norm) umgerechnet. In dem hier beschriebenen Beispiel wurde der Protein C Test auf dem Sysmex CS-5100 Analysegerät (Siemens Healthcare, Marburg, Deutschland) automatisch abgearbeitet.

Von allen der unter 2a) hergestellten Proben wurde eine Teilmenge zur Messung von Protein C verwendet (Testergebnis 1). Eine zweite Teilmenge jeder der unter 2a) hergestellten Proben wurde für 1 Stunde bei 133.000×g zentrifugiert, und die Lipid-abgereicherte Phase wurde für die Protein C-Messung als Probe eingesetzt (Testergebnis 2). Sofern die Zentrifugation keinen Einfluss hat, sollte die Lipid-abgereicherte Phase des zentrifugierten Aliquots dieselbe Analytmenge wie die nicht zentrifugierte Probe enthalten. Die beiden Testergebnisse (% der Norm) jeder Probe wurden miteinander verglichen, und es wurde die relative Differenz in % gebildet (100 × Testergebnis 1 - Testergebnis 2 / Testergebnis 2).

Aus den relativen Differenzen der Testergebnisse für die nicht-lipämischen Proben wurde der Mittelwert berechnet (hier: -2,4). Da die nicht-lipämischen Proben keine Interferenz durch Lipämie aufweisen, wird diese mittlere Differenz auf einen unspezifischen Einflussfaktor des Verfahrens (z.B. durch die Zentrifugation) zurückgeführt. Zur Korrektur dieses Effektes wurde für jede Probe eine korrigierte Differenz ermittelt, indem dieser Mittelwert von der relativen Differenz abgezogen wurde. Die korrigierte Differenz ist ein Maß für die durch Lipide verursachte Interferenz.

Tabelle 6 zeigt für jede Probe die Testergebnisse 1 und 2, die ermittelte relative Differenz und die korrigierte Differenz. Bei den Differenzwerten handelt es sich um gerundete Werte.

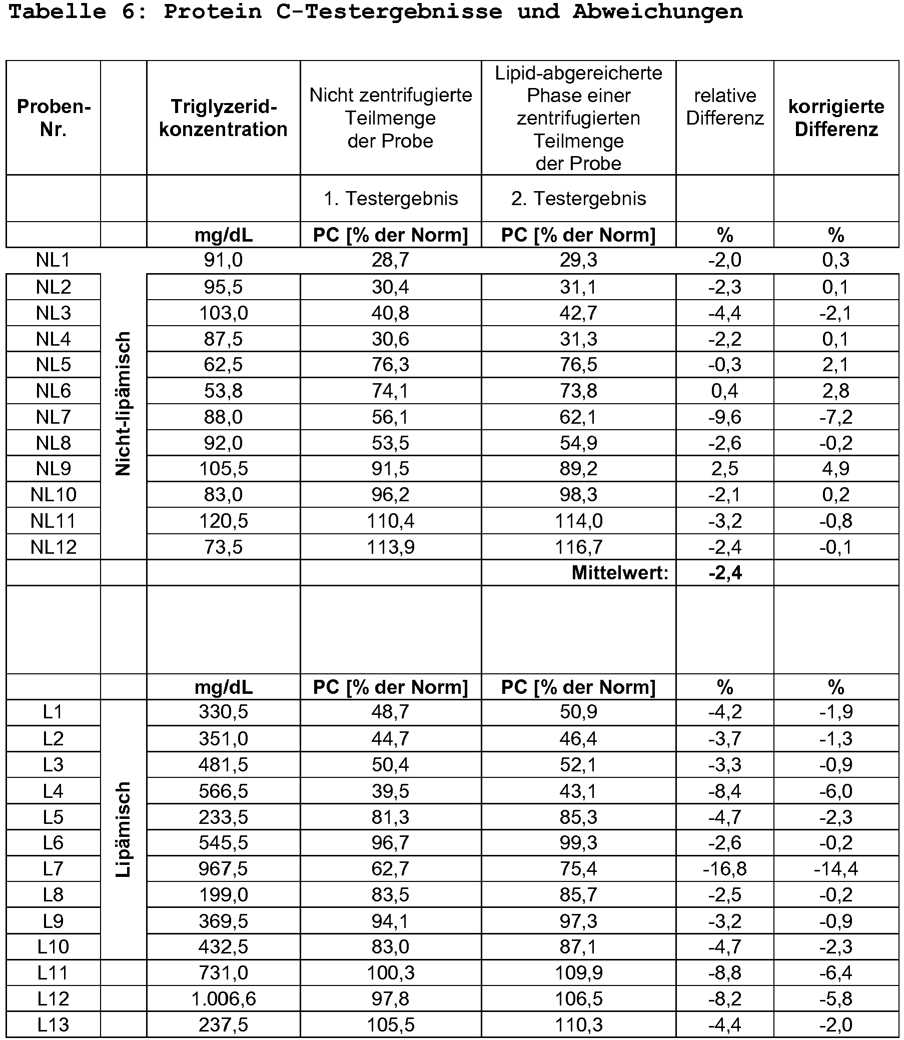

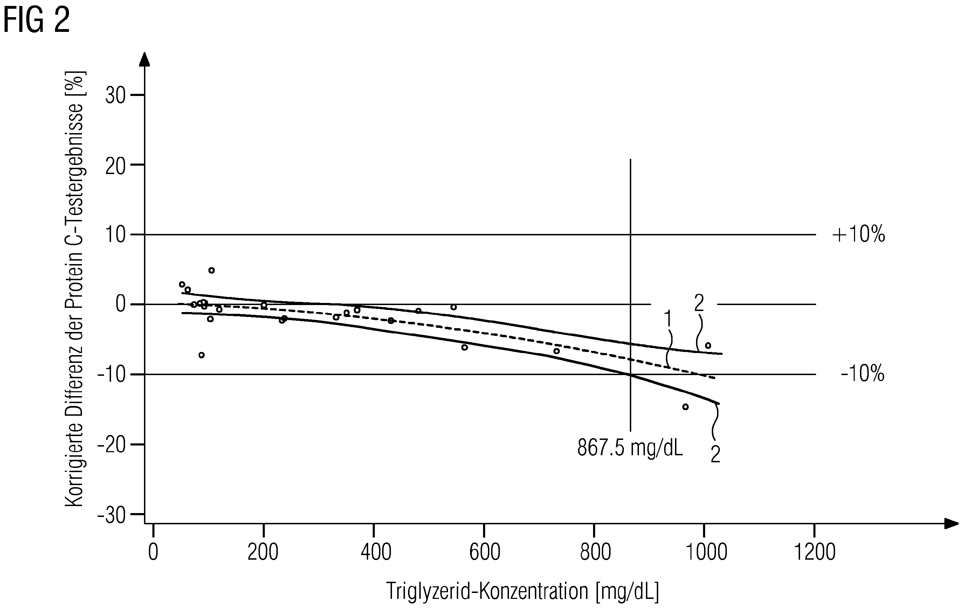

Die korrigierte Differenz, also die Abweichung der Testergebnisse der nicht zentrifugierten Proben von den Testergebnissen der zentrifugierten Proben(vermindert um den Mittelwert der Differenz der nicht-lipämischen Proben), wurde gegen die Triglyzeridkonzentration der nicht zentrifugierten Proben aufgetragen (Figur 2, Linie 1). Ein polynomisches Fitverfahren wurde angewandt und der dazugehörige Vertrauensbereich berechnet (Figur 2, Linien 2). Der Schnittpunkt der Grenze des Vertrauensbereiches mit dem Kriterium von 10 % relativer Abweichung kennzeichnet die Triglyzeridkonzentration, ab der eine Lipidinterferenz in dem getesteten Protein C-Testverfahren zu erwarten ist (867,5 mg/dL).

## Patentansprüche

1. Verfahren zur Herstellung einer lipämischen Plasma- oder Serumprobe, das Verfahren umfassend die Schritte:
(a) Zentrifugation einer ersten Teilmenge einer lipidhaltigen Plasma- oder Serumprobe zur Trennung eines lipidhaltigen Überstandes von einer Lipid-abgereicherten Phase;
(b) Abtrennung des lipidhaltigen Überstandes und
(c) Vermischen des lipidhaltigen Überstandes mit einer zweiten Teilmenge derselben lipidhaltigen Plasma- oder Serumprobe.

2. Verfahren gemäß Anspruch 1, wobei in (c) alternativ der abgetrennte lipidhaltige Überstand mit einer Teilmenge der Lipid-abgereicherten Phase vermischt wird.

3. Verfahren gemäß einem der vorherigen Ansprüche, wobei in Schritt (a) die lipidhaltige Plasma- oder Serumprobe für mindestens 10 Minuten bei mindestens 2000×g zentrifugiert wird.

4. Verwendung einer mit einem Verfahren gemäß einem der Ansprüche 1 bis 3 hergestellten lipämischen Plasma- oder Serumprobe in einem Verfahren zur Feststellung einer Lipidinterferenz in einem verfahren zur quantitativen Bestimmung der Menge oder der Aktivität eines Analyten in einer Plasma- oder Serumprobe.

5. Verfahren zur Feststellung einer Lipidinterferenz in einem Verfahren zur quantitativen Bestimmung der Menge oder der Aktivität eines Analyten in einer Plasma- oder Serumprobe, das Verfahren zur Feststellung einer Lipidinterferenz umfassend die Schritte:
(a) Bereitstellen eines ersten Testansatzes durch Vermischen mindestens eines analytspezifischen Nachweisreagenzes mit einer nicht-lipämischen Plasma- oder Serumprobe mit einer Analytkonzentration oder - aktivität und Messen eines ersten Testergebnisses;
(b) Bereitstellen eines zweiten Testansatzes durch Vermischen desselben mindestens einen analytspezifischen Nachweisreagenzes mit einer lipämischen Plasma- oder Serumprobe derselben Analytkonzentration oder -aktivität und Messen eines zweiten Testergebnisses;
(c) Feststellen einer Differenz zwischen dem ersten und zweiten Testergebnis; und
(d) Feststellen einer Lipidinterferenz, wenn die Differenz zwischen dem ersten und zweiten Testergebnis eine vorbestimmte Toleranzgrenze überschreitet;
**dadurch gekennzeichnet, dass** die lipämische Plasma- oder Serumprobe mit einem Verfahren gemäß einem der Ansprüche 1 bis 3 hergestellt ist.

6. Verfahren gemäß Anspruch 5, wobei die nicht-lipämische Plasma- oder Serumprobe mit einem Verfahren umfassend die folgenden Schritte hergestellt ist:
(a) Zentrifugation einer lipidhaltigen Plasma- oder Serumprobe und Isolierung der Lipid-abgereicherten Phase vom lipidhaltigen Überstand.

7. Verfahren gemäß einem der Ansprüche 5 und 6, wobei die lipämische Plasma- oder Serumprobe und die nicht-lipämische Plasma- oder Serumprobe aus derselben lipidhaltigen Plasma- oder Serumprobe als Ausgangsmaterial hergestellt sind.

8. Verfahren gemäß einem der Ansprüche 5 bis 7, wobei die nicht-lipämische und die lipämische Plasma- oder Serumprobe jeweils eine gegenüber der Norm verminderte oder erhöhte Analytkonzentration oder -aktivität aufweisen.

9. Verfahren zur Feststellung einer Lipidinterferenz in einem Verfahren zur quantitativen Bestimmung der Menge oder der Aktivität eines Analyten in einer Plasma- oder Serumprobe, das Verfahren zur Feststellung einer Lipidinterferenz umfassend die Schritte:
(a) Bereitstellen eines ersten Testansatzes durch Vermischen mindestens eines analytspezifischen Nachweisreagenzes mit einer ersten Teilmenge einer nicht-lipämischen Plasma- oder Serumprobe und Messen eines ersten Testergebnisses;
(b) Bereitstellen eines zweiten Testansatzes durch Vermischen desselben mindestens einen analytspezifischen Nachweisreagenzes mit der Lipid-abgereicherten Phase einer zweiten Teilmenge derselben nicht-lipämischen Plasma- oder Serumprobe, die zuvor für mindestens 10 Minuten bei mindestens 2000g zentrifugiert worden war, und Messen eines zweiten Testergebnisses; und
(c) Feststellen einer ersten Differenz zwischen dem ersten und zweiten Testergebnis; und
(d) Bereitstellen eines dritten Testansatzes durch vermischen desselben mindestens einen analytspezifischen Nachweisreagenzes mit einer ersten Teilmenge einer lipämischen Plasma- oder Serumprobe und Messen eines dritten Testergebnisses;
(e) Bereitstellen eines vierten Testansatzes durch Vermischen desselben mindestens einen analytspezifischen Nachweisreagenzes mit der Lipid-abgereicherten Phase einer zweiten Teilmenge derselben lipämischen Plasma- oder Serumprobe, die zuvor für mindestens 10 Minuten bei mindestens 2000g zentrifugiert worden war, und Messen eines vierten Testergebnisses; und
(f) Feststellen einer zweiten Differenz zwischen dem dritten und vierten Testergebnis; und
(g) Feststellen einer Lipidinterferenz, wenn die Abweichung zwischen der ersten und zweiten Differenz eine vorbestimmte Toleranzgrenze überschreitet,
**dadurch gekennzeichnet, dass** die lipämische Plasma- oder Serumprobe mit einem Verfahren gemäß einem der Ansprüche 1 bis 3 hergestellt ist.

## Claims

1. Method for preparing a lipemic plasma or serum sample, the method comprising the steps:
(a) centrifuging a first subamount of a lipid-containing plasma or serum sample in order to separate a lipid-containing supernatant from a lipid-depleted phase;
(b) removing the lipid-containing supernatant and
(c) mixing the lipid-containing supernatant with a second subamount of the same lipid-containing plasma or serum sample.

2. Method according to Claim 1, wherein in (c) alternatively the removed lipid-containing supernatant is mixed with a subamount of the lipid-depleted phase.

3. Method according to either of the preceding claims, wherein the lipid-containing plasma or serum sample is centrifuged in step (a) for at least 10 minutes at at least 2000 x g.

4. Use of a lipemic plasma or serum sample prepared using a method according to any of Claims 1 to 3 in a method for establishing a lipid interference in a method for quantitatively determining the amount or the activity of an analyte in a plasma or serum sample.

5. Method for establishing a lipid interference in a method for quantitatively determining the amount or the activity of an analyte in a plasma or serum sample, the method for establishing a lipid interference comprising the steps:
(a) providing a first assay mix by mixing at least one analyte-specific detection reagent with a nonlipemic plasma or serum sample having an analyte concentration or activity and measuring a first assay result;
(b) providing a second assay mix by mixing the same at least one analyte-specific detection reagent with a lipemic plasma or serum sample having the same analyte concentration or activity and measuring a second assay result;
(c) establishing a difference between the first and second assay result; and
(d) establishing a lipid interference when the difference between the first and second assay result exceeds a predetermined tolerance limit;
**characterized in that** the lipemic plasma or serum sample has been prepared using a method according to any of Claims 1 to 3.

6. Method according to Claim 5, wherein the nonlipemic plasma or serum sample has been prepared using a method comprising the following steps:
(a) centrifuging a lipid-containing plasma or serum sample and isolating the lipid-depleted phase from the lipid-containing supernatant.

7. Method according to either of Claims 5 and 6, wherein the lipemic plasma or serum sample and the nonlipemic plasma or serum sample have been prepared from the same lipid-containing plasma or serum sample as starting material.

8. Method according to any of Claims 5 to 7, wherein the nonlipemic and the lipemic plasma or serum sample have in each case an analyte concentration or activity that is reduced or elevated with respect to the norm.

9. Method for establishing a lipid interference in a method for quantitatively determining the amount or the activity of an analyte in a plasma or serum sample, the method for establishing a lipid interference comprising the steps:
(a) providing a first assay mix by mixing at least one analyte-specific detection reagent with a first subamount of a nonlipemic plasma or serum sample and measuring a first assay result;
(b) providing a second assay mix by mixing the same at least one analyte-specific detection reagent with the lipid-depleted phase of a second subamount of the same nonlipemic plasma or serum sample, which had previously been centrifuged at at least 2000 g for at least 10 minutes, and measuring a second assay result; and
(c) establishing a first difference between the first and second assay result; and
(d) providing a third assay mix by mixing the same at least one analyte-specific detection reagent with a first subamount of a lipemic plasma or serum sample and measuring a third assay result;
(e) providing a fourth assay mix by mixing the same at least one analyte-specific detection reagent with the lipid-depleted phase of a second subamount of the same lipemic plasma or serum sample, which had previously been centrifuged at at least 2000 g for at least 10 minutes, and measuring a fourth assay result; and
(f) establishing a second difference between the third and fourth assay result; and
(g) establishing a lipid interference when the deviation between the first and second difference exceeds a predetermined tolerance limit,
**characterized in that** the lipemic plasma or serum sample has been prepared using a method according to any of Claims 1 to 3.

## Revendications

1. Procédé de préparation d'un échantillon lipémique de sérum ou de plasma, le procédé comprenant les stades :
(a) centrifugation d'une première quantité partielle d'un échantillon lipidique de plasma ou de sérum pour séparer un surnageant lipidique d'une phase appauvrie en lipides;
(b) séparation du surnageant lipidique et
(c) mélange du surnageant lipidique à une deuxième quantité partielle du même échantillon lipidique de plasma ou de sérum.

2. Procédé suivant la revendication 1, dans lequel dans (c) en variante, on mélange le surnageant lipidique séparé à une quantité partielle de la phase appauvrie en lipides.

3. Procédé suivant l'une des revendications précédentes, dans lequel, dans le stade (a), on centrifuge l'échantillon lipidique de plasma ou de sérum pendant au moins 10 minutes à au moins 2000×g.

4. Utilisation d'un échantillon lipémique de plasma ou de sérum préparé suivant l'une des revendications 1 à 3, dans un procédé de constatation d'une interférence lipidique dans un procédé de détermination quantitative de la quantité ou de l'activité d'un analyte dans un échantillon de plasma ou de sérum.

5. Procédé de constatation d'une interférence lipidique dans un procédé de détermination quantitative de la quantité ou de l'activité d'un analyte dans un échantillon de plasma ou de sérum, le procédé de constatation d'une interférence lipidique comprenant les stades :
(a) on se procure une première composition de test en mélangeant au moins un réactif de détection spécifique à l'analyte à un échantillon non lipémique de plasma ou de sérum, ayant une concentration ou une activité d'analyte et on mesure un premier résultat de test;
(b) on se procure une deuxième composition de test en mélangeant le même au moins un réactif de détection spécifique à l'analyte à un échantillon lipémique de plasma ou de sérum de la même concentration ou activité d'analyte et on mesure un deuxième résultat de test;
(c) on constate une différence entre le premier et le deuxième résultat de test et
(d) on constate une interférence lipidique si la différence entre le premier et le deuxième résultats de test dépasse une limite de tolérance définie à l'avance;
**caractérisé en ce que** l'on prépare l'échantillon lipémique de plasma ou de sérum par un procédé suivant l'une des revendications 1 à 3.

6. Procédé suivant la revendication 5, dans lequel l'échantillon de plasma ou de sérum non lipémique est préparé par un procédé comprenant les stades suivants :
(a) on centrifuge un échantillon lipidique de plasma ou de sérum et on isole la phase appauvrie en lipides du surnageant lipidique.

7. Procédé suivant l'une des revendications 5 et 6, dans lequel on prépare l'échantillon lipémique de plasma ou de sérum et l'échantillon de plasma ou de sérum non lipémique, à partir du même échantillon lipidique de plasma ou de sérum, comme matière de départ.

8. Procédé suivant l'une des revendications 5 à 7, dans lequel les échantillons de plasma ou de sérum non lipémique et lipémique ont, respectivement, une concentration ou une activité d'analyte plus petite ou plus grande que la norme.

9. Procédé de constatation d'une interférence lipidique dans un procédé de détermination quantitative de la quantité ou de l'activité d'un analyte dans un échantillon de plasma ou de sérum, le procédé de constatation d'une interférence lipidique comprenant les stades :
(a) on se procure une première composition de test en mélangeant au moins un réactif de détection spécifique à l'analyte à un échantillon non lipémique de plasma ou de sérum, ayant une concentration ou une activité d'analyte et on mesure un premier résultat de test;
(b) on se procure une deuxième composition de test en mélangeant le même au moins un réactif de détection spécifique à l'analyte à un échantillon lipémique de plasma ou de sérum de la même concentration ou activité d'analyte et on mesure un deuxième résultat de test;
(c) on constate une différence entre le premier et le deuxième résultat de test et
(d) on se procure une troisième composition de test en mélangeant le même au moins un réactif de détection spécifique à l'analyte à une première quantité partielle d'un échantillon lipémique de plasma ou de sérum et on mesure un troisième résultat de test;
(e) on se procure une quatrième composition de test en mélangeant le même au moins un réactif de détection spécifique à l'analyte à une phase appauvrie en lipides d'une deuxième quantité partielle du même échantillon lipémique de plasma ou de sérum, qui a été centrifugé auparavant pendant au moins 10 minutes à au moins 2000g et on mesure un quatrième résultat de test et
(f) on constate une deuxième différence entre le troisième et le quatrième résultats de test et
(g) on constate une interférence lipidique si l'écart entre la première et la deuxième différence dépasse une limite de tolérance définie à l'avance,
caractérisé en que l'échantillon lipémique de plasma et de sérum est préparé par un procédé suivant l'une des revendications 1 à 3.
